# EUROPEAN PATENT SPECIFICATION

(11) **EP 0 474 797 B1**
(45) Date of publication and mention of the grant of the patent: **06.09.1995**
(21) Application number: 90915880.0
(22) Date of filing: 01.06.1990
(51) Int. Cl.: C12N 15/49, C12N 15/85, A61K 39/21, C12P 21/08, G01N 33/569, C12N 5/10

(54) **PROTEIN HAVING A TRANSACTIVATING PROPERTY, VECTORS EXPRESSING THIS PROTEIN, CELL LINES, AND USES THEREOF**
PROTEIN MIT TRANSAKTIVIERENDER EIGENSCHAFT, DIESES PROTEIN EXPRIMIERENDE VEKTOREN, ZELLINIEN UND SEINE VERWENDUNGEN
PROTEINE PRESENTANT UNE PROPRIETE DE TRANSACTIVATION, VECTEURS EXPRIMANT CETTE PROTEINE, LIGNEES CELLULAIRES, ET LEURS UTILISATIONS

(30) Priority: 02.06.1989 US 361028
(43) Date of publication of application: 18.03.1992
(73) Proprietor: DANA FARBER CANCER INSTITUTE, Boston Massachusetts 02115 (US)
(72) Inventor: HASELTINE, William, A., Cambridge, MA 02140 (US); TERWILLIGER, Ernest, Boston, MA 02215 (US); COHEN, Eric, Brighton, MA 02135 (US)
(74) Representative: Davies, Jonathan Mark
(86) International application number: US9003126
(87) International publication number: WO9015875

(56) References cited:
- US-A- 4 755 457
- AIDS RESEARCH AND HUMAN RETROVIRUSES, vol. 3, no. 1, 1987, pages 33-39, Mary Ann Liebert, Inc., Publishers; F. WONG-STAAL et al.: "Human immunodeficiency virus: The eighth gene"
- JOURNAL OF ACQUIRED IMMUNE DEFICIENCY SYNDROMES, vol. 3, no. 1, January 1990, pages 11-18, Raven Press, Ltd, New York, US; E.A. COHEN et al.: "Identification of HIV-1 vpr product and function"
- J. Virology, Volume 64, No. 6, issued 01 June 1990, (received U.S. Patent and Trademark Office 30 May 1990) E.A. COHEN, et al. "Human immunodeficiency virus vpr product is a virion-associated regulatory protein", pages 3097-3099.
- Nature, Volume 313, issued 24 January 1985, (London, GB), L. RATNER, et al. "Complete nucleotide sequence of the AIDS virus, HTLV-III", pages 277-283.
- Science, Volume 229, issued 05 July 1985. S. ARYA, et al. "Trans-activator gene of human T-lymphotropic virus type III(HTLV-III)" pp. 69-73.
- Biological Abstracts, Volume 89, No. 7 issued 01 April 1990, P. REISS, et al. "Antibody response to viral proteins U(vpu) and R (vpr) in HIV-1-infected individuals" see page AB-649, column 1, Abstract No. 72207, J. Acquired Immune Defic. Syndr., 3(2), 115-122.
- Biological Abstracts, Volume 89, No. 5, issued 01 March 1990, E.A. COHEN, et al. "Identification of HIV-1 vpr product and function" see page AB-436, column 2, Abstact No. 48611, J. Acquired Immune Defic. Syndr., 3(1), 11-18.

## Description

The present invention is directed to a new purified polypeptide, a vector expressing this polypeptide, the gene for this polypeptide a method of producing, this polypeptide, and use of this polypeptide. In particular, this gene and gene product can be used to increase the expression of desired gene products.

The human immunodeficiency virus (HIV-I, also referred to as HTLV-III, LAV or HTLV-III/LAV) is the etiological agent of the acquired immune deficiency syndrome (AIDS) and related disorders. [Barre-Sinoussi, et al., Science 220:868-871 (1983): Gallo et al. Science 224:500-503 (1984); Levy et al, Science 225:840-842 (1984); Popovic et al. Science 224:497-500 (1984); Sarngadharan et al, Science 224:506-508 (1984); Siegal et al, N. Engl. J. Med. 305:1439-1444 (1981)]. This disease is characterized by a long asymptomatic period followed by the progressive degeneration of the immune system and the central nervous system. Studies of the virus indicate that replication is highly regulated, and both latent and lytic infection of the CD4 positive helper subset of T-lymphocytes occur in tissue culture. [Zagury et al, Science 231:850-853 (1986)]. The expression of the virus in infected patients also appears to be regulated as the titer of infectious virus remains low throughout the course of the disease. Molecular studies of the replication and genomic organization of HIV-I show that it encodes a number of genes [Ratner et al, Nature 313:277-284 (1985); Sanchez-Pescador et al, Science 227:484-492 (1985); Muesing et al, Nature 313:450-457 (1985); Wain-Hobson et al, Cell 40:9-17 (1985)]. Three of the genes, the gag, pol and env genes are common to all retroviruses. However, the genome also encodes additional genes that are not common to most retrovirus, the tat, rev (formerly referred to as art), nef, vif, and vpu genes [Sodroski et al, Science 231:1549-1553, (1986); Arya et al, Science 229: 69-73 (1985); Sodroski et al, Science 227:171-173 (1985): Sodroski et at, Nature 321:412-417 (1986); Feinberg et al, Cell 46:807-817 (1986); Haseltine, W.A., Journal of Acquired Immune Deficiency Syndrome. 1:217-240 (1988); Cohen, E. et al, Nature 334:532-534 (1988) which are all incorporated herein by reference.] Another gene has been identified, vpr, but its funciton was unknown [Wong-Staal, F., et al, AIDS Res. and Human Retro Viruses 3:33-39 (1987); which is incorporated herein by reference].

Nucleotide sequences from viral genomes of other retroviruses, namely HIV-II and Simian immunodeficieny virus (SIV) (previously referred to as STLV-III) also contain tat, rev and nef regulatory sequences and show transactivation in addition to containing the structural genes. [Guyader et at, Nature 326:662-669 (1987); Chakrabarti et at, Nature 328:543-547 (1987)].

The discovery of a functional gene, its expression products and their properties are important to understanding the viral life cycle.

In addition, it is desirable to be able to have a gene and/or gene product that can be used in a wide range of expression systems to increase the expression of a desired product.

### Summary of Invention

We have now discovered a new protein that can be used to increase the expression of a desired product in a wide range of expression systems. This protein contains a sufficient number of amino acids corresponding to amino acids 
MEQAPEDQGPQREPHNEWTLRLLEELKNEAVRHFPRIWLHGLGQHIYETYGDTWAGAEAIIRILQQLLFIHF
RIGCQHSRIGVTQQRRARNGASRS
to provide a transactivation function and is referred to as viral protein R or rap protein. This protein has a molecular weight of about 15kD. This protein is expressed by an "active" gene. Such an active vpr gene preferably corresponds to a sufficient number of nucleotides corresponding to the R open reading frame of HIV strains BRU, MAL and ELI to encode a protein having a transactivating property.

It is possible to construct an expression vector that contains a nucleotide segment corresponding to a sufficient number of nucleotides corresponding to the R open reading frame of HIV strains BRU, MAL and ELI to encode a protein having a transactivating property. This vector does not contain a sufficient number of nucleotides corresponding to an entire HIV genome. Preferably, the vector consists essentially of this nucleotide segment and the sequences necessary to express the rap protein.

The vpr vector can be used to transfect a cell line to create stable vpr cells.

The above-described vpr vector can be used to increase the production of a desired heterologous gene product. This can be accomplished by transfecting a pre-selected cell line that contains the desired heterologous gene product with the vpr vector, or by co-transfecting a preselected cell line with the vpr vector and with an expression vector containing the desired heterologous gene or by using a vpr vector that also contains the desired heterologous gene product and transfecting a pre-selected cell line with that vector. Alternatively, one can use an expression vector containing the desired heterologous gene to transfect a vpr cell line to result in the increased production of the heterologous gene.

### Brief Description of The Drawings

Figure 1 is a schematic showing the location of the vpr gene in the HIV genome.

Figure 2 is a schematic showing the construction of transcription expressors derived from HIV strains, HXBc2, BH5, BH10, BRU and ELI.

Figure 3 is a schematic showing the construction of a nucleotide segment able to express active vpr protein.

Figure 4 is a schematic showing the size of predicted vpr proteins from published HIV sequences.

Figure 5 shows the predicted amino acid sequences of the vpr protein for a number of HIV strains.

Figure 6 are autoradiograms showing immunoprecipitation of ³⁵S met labeled protein from a number of HIV strains.

Figure 7 shows the construction of a plasmid that will express an active vpr and a second plasmid that will not express an active vpr.

Figure 8 is a comparison of the rate of cell growth and syncytia formation in cells transfected by HIV virus expressing active vpr (vpr⁺) and cells transfected by vpr⁻ isogenic virus.

Figure 9 is a graph showing the effect of active vpr protein on the expression of a heterologous gene.

Figure 10 is a graph showing the effect of active vpr protein on the expression of a heterologous gene.

Figure 11 are autoradiograms showing immunoprecipitation of ³⁵S cys labeled protein in virus supernatant over time.

Figure 12 are autoradiograms showing immunoprecipitation of ³⁵S cys labeled protein in cell lysate over time.

Figure 13 shows reverse transcriptase in culture transfected with vpr⁺ or vpr⁻ genes.

### Detailed Description of the Invention

We have now discovered an active form of the vpr protein, as well as a gene that expresses that active form.

This protein is expressed by a gene that corresponds to a sufficient number of nucleotides of the R open reading frame of an HIV or SIV strain, collectively referred to as HIV, to encode a viral protein having a transactivating effect. Preferably, the nucleotides correspond to the R open reading frame of HIV-1 strains BRU, MAL or ELI.

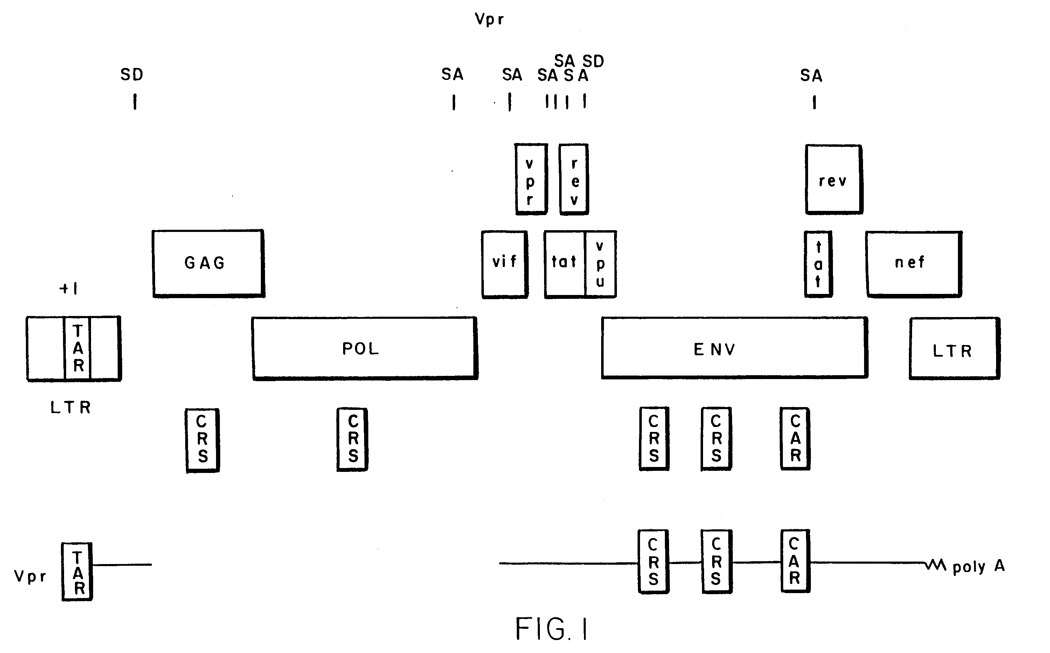

The vpr gene overlaps a portion of the vif gene and the first exon of the tat gene. See Figure 1. This gene can readily be excised from the HIV genome by taking advantage of known splice acceptor sites and standard techniques such as using restriction endonucleases. Alternatively, a synthetic nucleotide sequence can be prepared based upon this sequence.

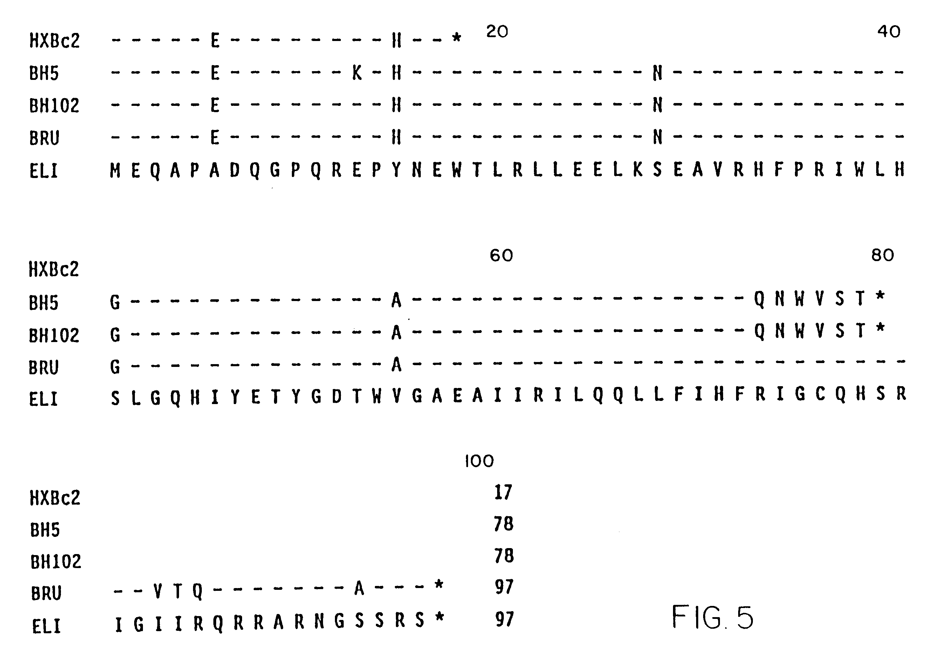

The protein having a transactivating property is typically a 15kD protein. This protein is referred to as active viral protein R or rap protein. Preferably, the rap protein has a sufficient number of amino acids corresponding to 
MEQAPEDQGPQREPHNEWTLRLLEELKNEAVRHFPRIWLHGLGQHIYETYGDTWAGAEAIIRILQQLLFIHF
RIGCQHSRIGVTQQRRARNGASRS
to have a transactivating function. Substitutions, deletions and/or additions of amino acids that do not effect the protein's transactivating property are referred to as the rap protein. For example, the proteins shown in Figure 5 for BRU and ELI both have a transactivating property, although there have been some substitutions of amino acids among the two strains. Similarly, deletion of the starting Met will not affect the rap protein's transactivity function. This property can readily be determined empirically. For example, by comparing the expression of a specific heterologous gene, preferably a marker gene, in a cell transfected with a nucleotide sequence believed to express functional rap protein and its expression in a cell transfected by an isogenic vector having a defective vpr sequence, such as one having a stop codon about 120 nucleotides into the R open reading frame. A functional rap protein will result in at least a doubling of expression of the heterologous gene when compared to its expression in the cell with the defective vpr sequence.

A vpr gene was reported by Wong-Staal, F. et al, in AIDS Res. and Human Retrovirus 3, supra. The existence of a protein specified by this region frame was inferred from the presence of a conserved open reading frame (called R) that spans this region and by the existence of antibodies in some HIV-I infected patients, which recognized a protein that was predicted to be specified by this open reading frame. Such antibodies were not found in the serum of all HIV-I infected patients. It was subsequently reported that a vpr gene sequence was present and intact in most HIV-I and HIV-II isolates and in the SIV_{MAC} isolate but not in the SIV_{AGM} isolate. Based upon reported nucleotide sequences Wong-Staal et al indicated that the BH10, BH5, HXB-2 and H9ₚᵥ strains contained a termination codon after amino acid 77 whereas, the BRU, the ARV, the HAT-3, the ELI, and the MAL strains could express an additional 18 or 19 amino acids as a result of a frameshift. It was also reported that the R gene from HXB-2 and BH10 should be sufficient to produce the viral protein. Based upon this report, individuals attempted to determine the funciton of the protein expressed by this gene. However, no function has to date been assigned to vpr. Using isogenic proviruses, except for the vpr sequences, no differences were found between vpr⁻ mutants and vpr⁺. For example, it was reported that vpr⁻ mutants replicated in CD4+ T cells at the same rate as the vpr⁺ viruses. Similarly, the vpr⁻ mutants were cytotoxic to CD4+ T cells in culture.

However, we have now found that the reported sequences for many of the strains apparently contain an error in the nucleotide sequences. Thus, while these strains were believed to produce an active form of the rap protein, they do not because they actually contain a premature termination codon in the R open reading frame. Accordingly, although many HIV strains appear to have the capacity to encode a functional protein in this region, they do not because they sustained mutations that prematurely terminate the protein.

Surprisingly, we have found that while not necessary for viral growth, the vpr protein has the ability to enhance the expression of proteins in-trans. Accordingly, we suggest that functional viral protein R be redesignated rap to refer both to its original designation (vpr and the R open reading frame) and to indicate the phenotype conferred by this gene, i.e. rapid growth. Thus, the functional protein is sometimes referred to as the rap protein. The gene expressing the functional protein is referred to as an active rap gene or an active vpr gene.

A vector designed to express an active vpr protein will contain a nucleotide segment corresponding to a sufficient number of nucleotides corresponding to the R open reading frame to express a protein having a transactivating function. The nucleotides can correspond to R regions from HIV-1, HIV-II or SIV (collectively referred to as HIV). Preferably, the nucleotides correspond to the R open reading frame of the BRU, MAL or ELI strains of HIV-1. This vector will not contain nucleotides corresponding to the entire HIV or SIV genomes. Preferably, the vector will not contain nucleotides corresponding to at least one of the following--the second exons of rev or tat, a functional env, a functional pol, or a functional vif. In one preferred embodiment, the nucleotides corresponding to nucleotides from the HIV and SIV genome will consist essentially of those corresponding to an active vpr gene. The nucleotides can be RNA or DNA, but are preferably DNA.

The vpr vector can be prepared by standard techniques well known to the art. For example, a vpr vector can be constructed containing an active vpr gene, polyadenylation sequences downstream (3') from the gene and a promoter to permit expression of the vpr gene. For example, one can use known expression vectors and insert the nucleotide segment containing an active vpr gene into that vector by use of appropriate splice acceptor and donor sites and the appropriate restriction endonucleases. Preferably, the vector also contains a replication origin. Promoters contained in the vector can be any of the known promoters and the choice is governed by the host cell one wishes to use. Such promoters will include retroviral promoters, such as AKV, SL3-3 and Friend viruses. The use of a retroviral promoter is preferred. The vector can also contain an enhancer. Such enhancers are known in the art, for example, a viral enhancer. Preferably, the vector contains an enhancer which is tissue specific. More preferably, the enhancer is of the same class as the promoter.

As aforesaid, the functional vpr protein produced by this vector has the ability to accelerate growth in trans. Thus, it can be used to stimulate the expression of a desired heterologous gene product, for example, the chloramphenicol acetyltransferase (CAT) gene product. The 15kD rap protein will stimulate expression of a desired heterologous gene that is on the same vector as it is. The rap protein will also stimulate expression of heterologous genes that are on vectors that are cotransfected with it, as well as heterologous genes that are already present in the cell. Alternatively, one can create stable rap cell lines and transfect those cell lines with a vector containing the desired heterologous gene. One cultures the transfected cells and then collects the desired protein. The particular method used to obtain the desired protein will depend upon what the particular desired protein being expressed is.

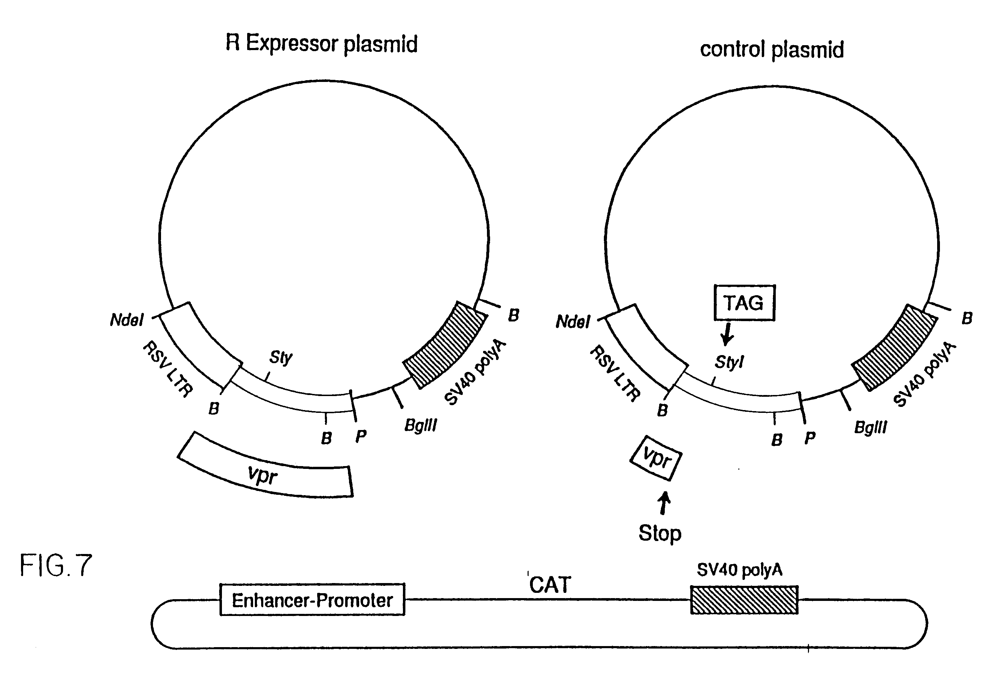

The functional rap protein has thus far stimulated the expression of a heterologous gene in a wide range of expression systems. Such systems included those having the expression of the heterologous gene under the control of retroviral, viral and cellular promoters. For example, a heterologous CAT gene, whose expression was under the direction of HIV-I LTR had enhanced expression ranging from 3 to 8 fold in several different cell lines compared to control cells where the CAT gene was co-transfected with rap defective genes. A vector containing an active vpr gene whose nucleotide sequence corresponded to a sufficient number of nucleotides from the ELI strain to express a functional rap protein, cloned 3' to the RSV LTR and cotransfected in Jurkat cell lines with a CAT expression vector (See Figure 7), when compared to a control vector, produced the results shown in Table 1 below.

**TABLE 1**

| STIMULATION OF GENE EXPRESSION BY VPR | | |
|---|---|---|
| Enhancer-promoter | -vpr | +vpr_{ELI} |
| (Retrovirus) | | |
| HIV-1 | 1 | 3-4 |
| HIV-2 | 1 | 3 |
| SL3 | 1 | 10 |
| RSV | 1 | 8 |
| HTLV-1 | 1 | 3 |

| (DNA Virus) | | |
|---|---|---|
| CMV | 1 | 3 |
| SV40 | 1 | 3 |

| (Cellular Regulatory Regions) | | |
|---|---|---|
| cfos | 1 | 2 |
| IL2-R-Hyb. | 1 | 5 |

As demonstrated by Table 1, the rap protein stimulates the effect of a heterologous gene's expression under the control of a wide variety of enhancer-promoters, in this example, viral (both retroviral and DNA viral) and cellular promoters with no cell-type specificity. We found that cotransfection by a vector containing a heterologous gene, such as CAT, and a rap vector of tat Jurkat cells, i.e., cells stably expressing the tat gene, still resulted in enhanced expression as a result of the rap protein, that vpr and tat transactivation was additive.

We expect that one would also obtain transactivation by the rap protein by adding purified rap protein to a cell, rather than cotransfection. This can be accomplished, for example, by adding the rap protein as a supplement to the culture medium. Purified rap protein can redily be obtained by using the rap vector to transform a cell and then purifying the rap protein by standard techniques. Purities of 90% or greater, and more preferably of 95% or greater can be obtained.

Various cell lines may differ in their ability to take up and express transfected vpr DNA. However, by use of appropriate promoters a wide range of cells can preferably be used. For example, Raji cells, HUT 78 cells, Jurkat cells, HeLa cells and NIH 3T3 cells are preferred. Human T-cells and B-cells generally are useful. Typically, mammalian cells would be preferred, but cell lines of the present invention are not limited thereto.

The vector can also contain a marker gene to aid in detection of transformed cells. This can include an antibiotic resistance gene such as the bacterial neomycin (neo) resistance gene that confers a dominant selectable resistance to the antibiotic G418 in eukaryotic cells [Southern and Berg, J. Mol. Appl. Genet. 1:327-341 (1982)]. The vector can be used to transform cell lines, for example by being introduced into psi/2 (ecotropic) and psi AM (amphotropic) cell lines, by the calcium phosphate co-precipitation method. [Wigler, et al, Cell 16:777-785 (1979)]. These cell lines can constitutively produce murine leukemia virus proteins that can not package the viral transcripts [Cone, et al, PNAS 81:6349-6353 (1984); Mann, et al, Cell 33:153-159 (1983)]. The vector can be modified so that transcripts from the vector containing the vpr sequences as well as the neomycin resistance sequences will be packaged within murine leukemia virus particles. Two days following transfection, cells can be selected by looking for the marker, i.e., using the antibiotic G418. G418 resistant clones are evident in 7 to 10 days. Insertion of the vpr exon does not interfere with splicing events required for transcription of neo genes. G418-resistant, psi2 and psiAM clones are isolated and clones producing large amounts of murine virus particles are selected and used to infect test cells. These cells are then cultured in a standard medium and harvested as needed.

Such cell lines would express the rap protein and one could enhance expression of a desired protein by expressing it in this stable rap-expressing cell lines.

The rap protein is immunogenic. By making an antibody corresponding to the C terminal most end, one should be able to differentiate between active rap protein and non-active rap protein. Other antibodies can also be made. For example, the N terminal most portion of the vpr gene is immunogenic and can be used to raise antibodies. The antibody generated can be polyclonal or monoclonal depending upon the particular application for which is is designed. Such antibodies can be prepared by established techniques well known to the skilled artisan. For example, an oligopeptide can be conjugated to keyhole limpet hemocyanin (KLH) and used to raise an antibody in an animal such as a rabbit. Typically, peptide-KLH conjugate is injected several times for a period of about two weeks to generate an antibody. The antibody is then collected from serum by standard techniques. Alternatively, monoclonal antibodies can be produced in cells which produce antibodies to the protein by using standard fusion techniques for forming hybridoma cells. Typically, this involves fusing an antibody producing cell with an immortal cell line, such as a myeloma call, to produce the hybrid cell.

Assays for the vpr protein can be prepared using standard techniques. For example, one can take a predetermined sample, i.e., the biological specimen to be tested, and add an antibody to the N-terminal portion of the vpr protein. One can preferably use monoclonal antibodies. This sample is then screened to determine if there is a reaction, i.e. if a complex is formed between antibody and antigen. Alternatively, one can assay with antibodies either monoclonal or polyvalent to the antigenic determinants of the viral protein itself using known immunoassay methods, for example, using competitive immunoassays or immunometric (sandwich) assays.

The present invention is further illustrated by the following examples. These examples are provided to aid in the understanding of the invention and are not to be construed as a limitation thereof.

### EXAMPLES

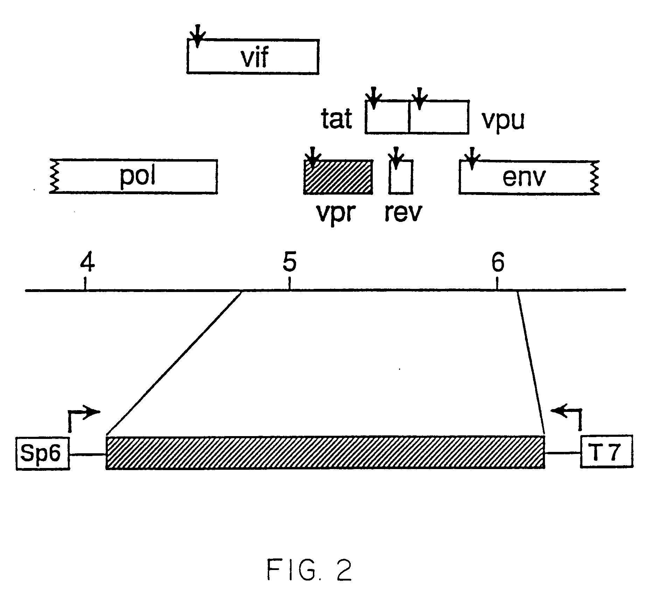

The ability of the vpr gene to encode an active protein was examined by programming an in vitro reticulocyte translation lysate [Pelham. H.P.B. et al. Eur. J. Biochem. 67: 247-256 (1986)] with RNA synthesized in vitro using the method of Melton. D.A. et al. Nucl. Acid Res. 12:7035-7056 (1984). The template for the experiment was derived from a fragment of the HXBc2 provirus [Arva et al. Science 229:69-73 (1985)] placed 3' to the SP6 bacteriophage RNA polymerase promoter. The viral sequence present in this RNA transcript, as shown in Figure 2, extends from the C-terminal of vif (StuI site at position 4993) to the N-terminal of env (KpnI site at position 5934). Internal restriction sites used to linearize the plasmids are indicated by arrows.

Proteins produced in the in vitro lysate using the RNA-derived fragment were labeled with ³⁵S-methionine and separated by size using SDS-PAGE.

The plasmid was linearized by digestion at an EcoRI site located in the polylinker 3' to the HIV_{HXBx2} insert and used as a template for in vitro transcription by SP6 RNA polymerase as described [Pelletier, J. and Sonenberg, N., Cell 40:515-526 (1985)] except that the concentration of GTP and Cap analog m⁷GpppG was raised to 0.2 and 1.0mM, respectively. Messenger RNAs were labeled with [5-³H]CTP and purified as described. In vitro translation of equimolar amounts of RNA (equal amounts of radioactivity) were performed in reticulocyte lysate [Pelham, H.P.B., et al, Eur. J. Biochem. 67:247-256 (1986)]. Incubation was done at 30°C for 30 minutes in the presence of ³⁵S-cysteine. Labeled products were analyzed directly by 15% SDS-PAGE or immunoprecipitated. [Lee, T.H., et al, Cell 44:941-947] beforehand with free immune rabbit serum.

Immunoprecipitation of the labeled lysate with an anti vpr anti serum was performed. The anti vpr anti serum was raised by using an oligopeptide corresponding to the first 19 amino acids of the N-terminal portion of the vpr amino acid sequence. This amino acid sequence corresponded to the vpr protein that the BRU substrain of the HIV is predicted to make. The resultant peptide was conjugated to keyhole limpet hemocyanin (KLH) and used to raise antibodies in three rabbits. Multiple injection of the antigens using standard techniques was made. Thereafter, the rabbits were shown to produce antibodies that recognized the oligopeptides.

The RNA made in vitro has the capacity to encode vpr, the first exon of tat and rev, and 40 amino acids of the env gene. Although this segment contains much of the vpu gene, it is not capable of expressing the vpu gene as it does not contain an intact vpu initiation codon.

We followed the above procedures with templates corresponding to the same section from other HIV viral strains. The viral strains used were BH5, BH10, ELI and BRU. The RNA made in vitro in these strains has the same capacity as that for HXBc2, except that some of these strains have an initiation codon for the vpu gene and thus also have the capacity to encode the vpu protein.

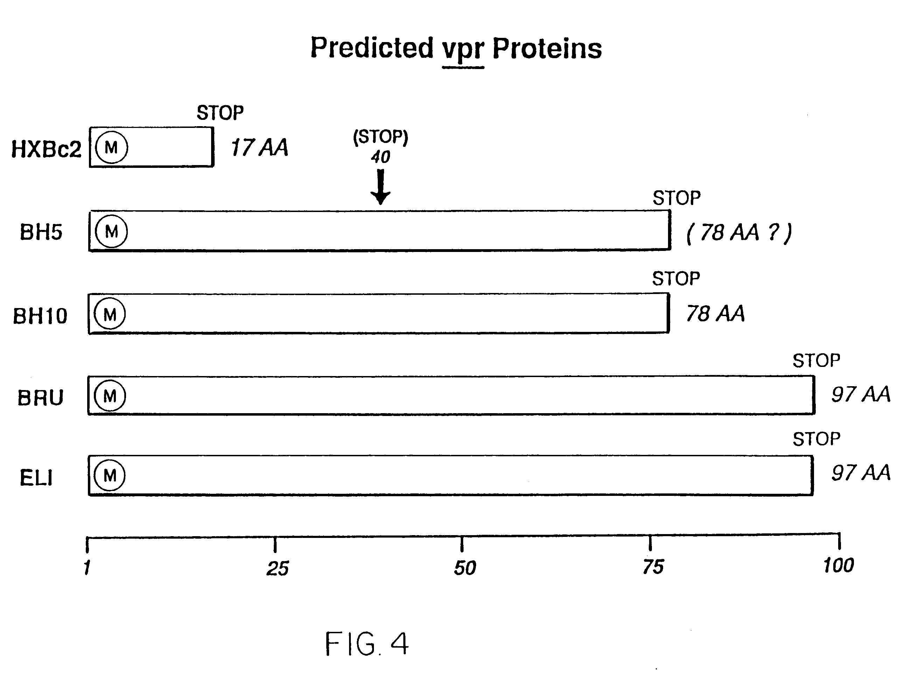

The predicted vpr proteins for these strains is shown in Figure 4. The predicted amino acid sequence of these proteins for these strains is shown in Figure 5. Dashes indicate identical amino acid sequences.

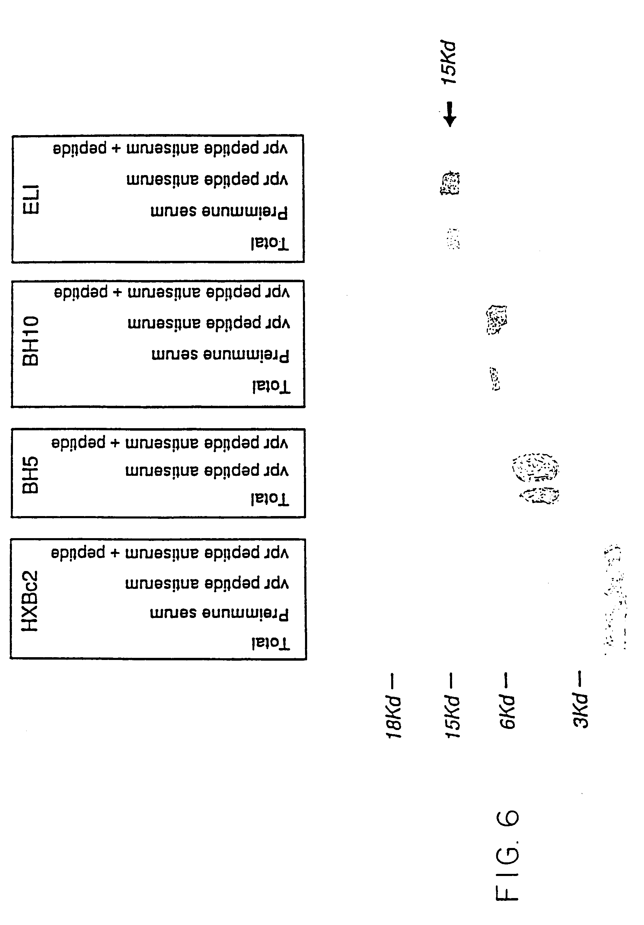

Translation of the RNA corresponding to the regions of the ELI, MAL, and BRU strains of HIV gave rise to a 15kD protein that was recognized by a anti vpr, antisera. Figure 6 shows autoradiograms of the product immunoprecipitated In strains HXBc2, BH5, BH10 and ELI, respectively. Lanes 1 for HXBc2, BH5, BH10 and ELI show total. Lanes 2 for HXBc2, BH10 and ELI show preimmune serum. Lane 2 for BH5 shows vpr peptide antiserum. Lanes 3 for HXBc2, BH10 and ELI show vpr peptide antiserum. Lane 3 for BH5 shows vpr peptide antiserum and synthetic peptide used to raise antisera. Lanes 4 for HXBc2, BH10 and ELI show vpr peptide antiserum and peptide. Lane 3 for ELI shows a 15kD peptide was produced. Translation of similar RNA, derived from BH10 and BH5 resulted in polypeptides of 8kD and 5kD (lanes 3 and 2, respectively). Translation of the RNA derived from HXBc2 resulted in no detectable protein (lane 3 of Figure 6). This data is not identical with that which would be predicted, based on the reported sequences. See Figures 4 and 5. We believe that it is likely chat the BH10 strain contains a premature termination codon in the vpr open reading frame. A comparison of the nucleotide sequences of the vpr gene of BH10, ELI, BRU and MAL shows an insertion of a T in the BH10 sequence, which would provoke a frameshift to another frame where a stop codon is present. Thus, although many strains of HIV-1 appear to have the capacity to encode a 15kD protein, we believe that many proviral clones do not actually have this ability.

We believe that the vpr protein of the HXBc2 provirus of the IIIB strain of HIV-1 is defective for expression of the 15kD protein by virtue of having two widely separated base changes that prevent expression of the protein. Similarly, we believe that the BH5 vpr gene also contains two point mutations which lead to premature termination of the protein contrary to the published sequence data. The in vitro translation product of BH5 seems faster than the 5kD marker, thus indicating the existence of a probable stop codon upstream to the published one. Accordingly, we have surprisingly found that the BRU, the ELI and the MAL strains contrary to the report of Wong-Staal do not have a frameshift, nor do they express the same protein as the BH10, BH5 and HXBc2 strains, but rather, express a functional protein.

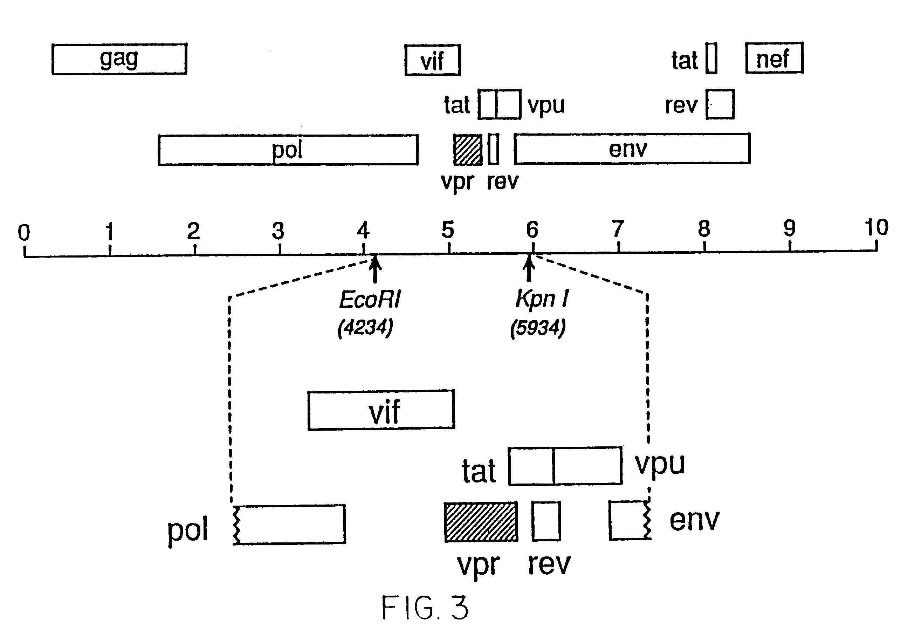

Pairs of isogenic proviruses, except for the vpr region, were constructed using the HXBc2 provirus as a backbone and a fragment containing the vpr gene from the BRU provirus. See Figure 3. The vpr region in the HXBc2 recombinant (HXBRU) extended from the C terminal of pol (EcoR1, 4234) to the N terminal of env of BRU (Kpn1 5934). See Figure 3. A NcoI frameshift mutation introducing a termination codon at amino acid 40, was introduced to generate the vpr⁻ isogenic recombinant, HXBRU vpr⁻.

All plasmids were made by standard procedures using restriction and modification enzymes according to manufacturers suggestions. The HIV transcripts were inserted into these vectors by standard techniques. Jurkat cells were transfected by the DEAE-dextrin procedure using 10µg of the plasmid to be tested.

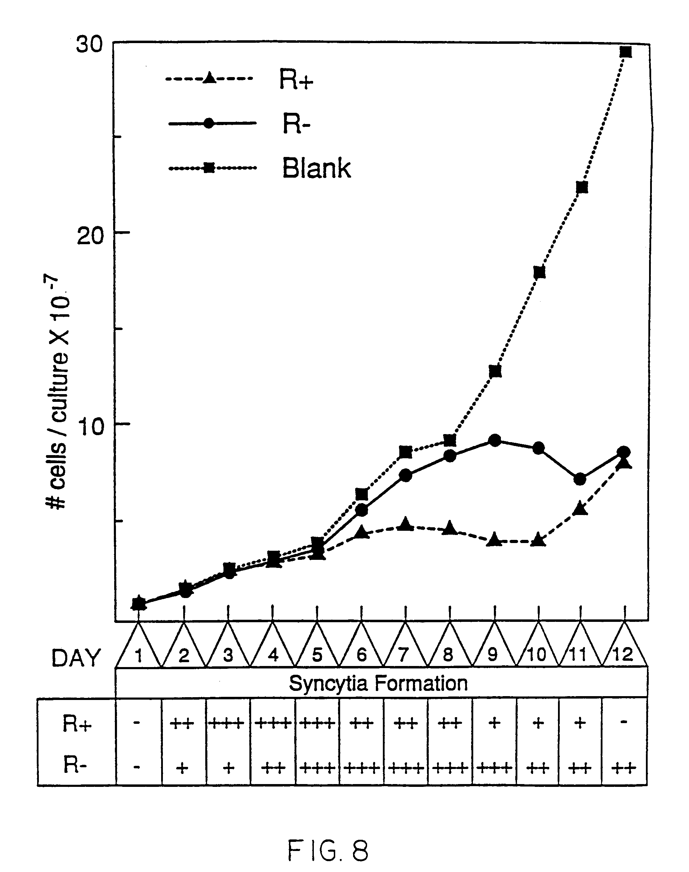

Cultures were then monitored for total cell number and the appearance of giant multinucleated cells, syncytia. A reproducable difference in the effect of virus replication on cell numbers was observed for vpr⁺ and vpr⁻ viruses. See Figure 8. In the graph on Figure 8, the lines with a box are the normal cells that have not been transfected with either provirus. The lines with a triangle indicate cell growth over time in cells transfected with the vpr provirus, whereas the lines with a circle indicate the cell growth in cells transfected with the vpr⁻ virus (HXBRU vpr⁻). In all cell cultures, the cell number rose normally until five days post transfection. For the next three days (day 8) the rate of increase in the cell number in culture transfected with the vpr⁺ provirus was markedly lower than that of the vpr⁻ transfected culture. By day 11, the total number of cells had decreased sharply in the culture infected with the vpr⁺ virus. By contrast, the total number of cells in the culture transfected with the vpr⁻ virus continued to rise on days 6 to 9.

A difference in the kinetics of appearance of large multinucleated giant cells was also detected in cultures infected with vpr⁺ and vpr⁻ viruses. Syncytia were first evident in culture infected with the vpr⁺ virus within two days post transfection and reached a maximum by day 3 to 5 with only small numbers of syncytia remaining by days 9 to 11. In contrast, the number of syncytia was maximal in culture infected with the vpr⁻ provirus between days 5 to day 9. By day 12 no syncytia remained in the culture infected with vpr⁺ virus. However, syncytia could still be observed in the culture infected with vpr⁻ provirus. This experiment indicates that active vpr gene and its gene product is not required for cytotoxic activity but does effect the timing of the cytotoxic effect.

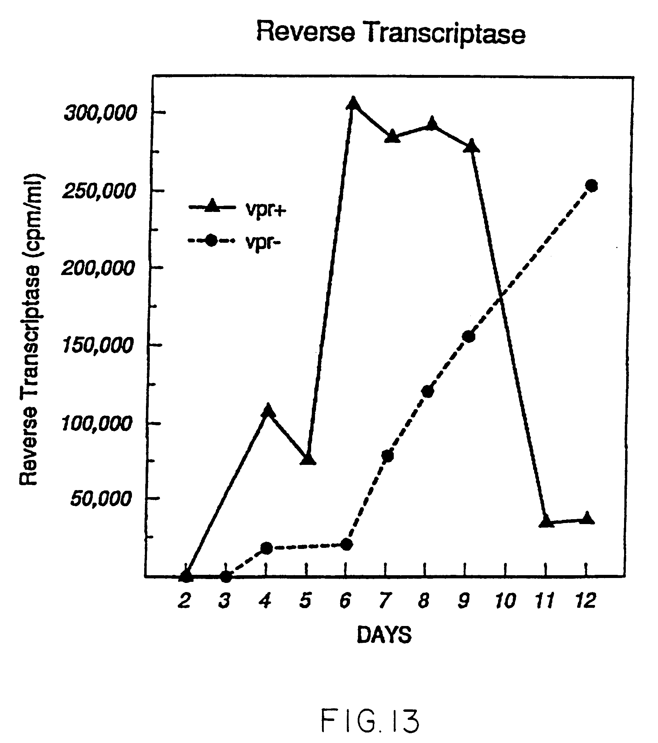

The effect of active vpr protein on production of virus particles was determined by the amount of virus released into the supernatant, as measured by the level of the major viral capsid protein, p24, and extracellular RT. Cells were pelleted and levels of viron associated RT and p24 present in the supernatant were determined using standard assay techniques. Figure 13 shows that in culture infected with the vpr⁺ gene, reverse transcriptase was detected by day 3 post transfection and reached a peak 3 to 4 days later, whereas virus production was severly decreased by day 12 and 13. In contrast, in the cells transfected with the vpr⁻ provirus, reverse transcriptase was detectable in the culture only at day 5. At day 12 and 13 there was still a considerable amount of virus produced. The data obtained from p24 assays closely paralled these results. Once again, there was a lag in production in the vpr⁻ provirus. In Figure 13, the line with the triangles represents culture with vpr⁺ gene, while the line with the circles represents cuclture with vpr⁻ gene.

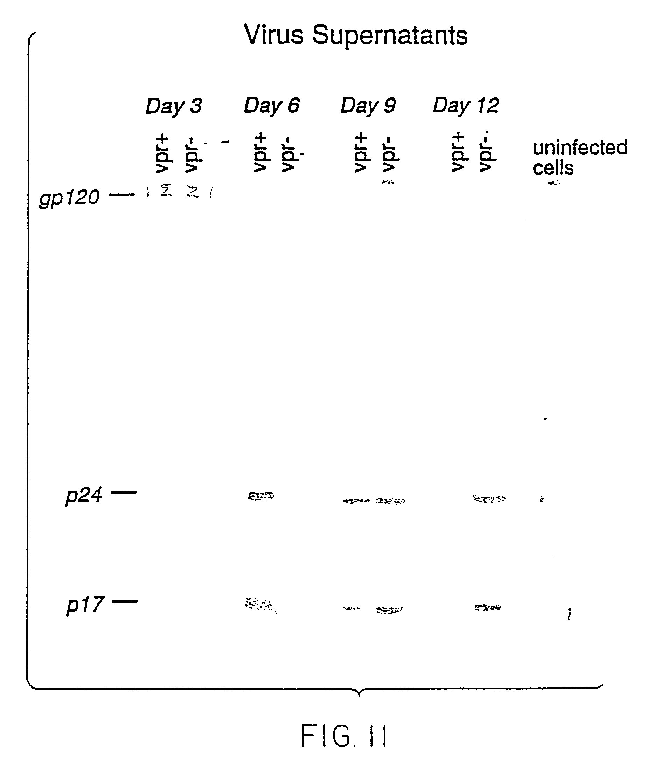

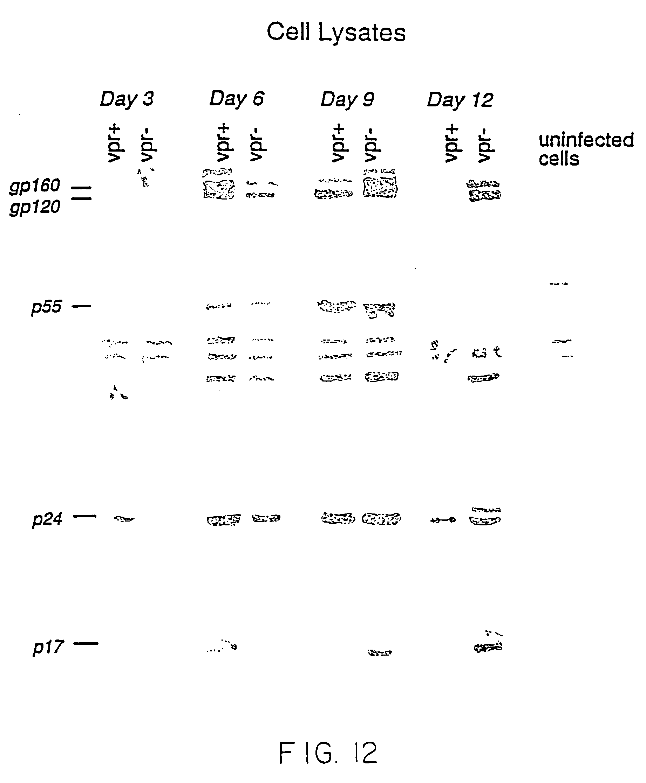

By immunoprecipitation of ³⁵S cysteine labeled protein with AIDS patient serum, a profile of expression of virus-specific proteins was determined at different days post transfection. See Figures 11 and 12. At day 3, gp160, gp120, p55, p24 and p17 can be immunoprecipitated from cells infected with the vpr⁺ provirus (HXBRU). No detectable viral protein was seen at day 3 in cells infected with the vpr⁻ provirus.

In both the cell lysate and the virus supernatant, more viral protein was made in the culture infected with the vpr⁺ provirus compared to the culture infected with the vpr⁻ provirus at an early point in time. Accordingly, these experiments indicate that the active vpr gene (vpr⁺) produces a protein that confers a rapid growth phenotype to the virus.

An active rap (vpr⁺) expression vector was prepared using. standard techniques. This vector contained a DNA segment containing nucleotides corresponding to a sufficient number of the vpr nucleotides of the ELI prostrain of HIV to express a functional rap protein. This fragment was cloned 3' to the RSV LTR in the plasmid shown in Figure 7. This plasmid also contains the SV40 polyadenylation signals. The vpr sequence was inserted into the expression vector at the sites shown. A virtually identical expression vector, except for the substitution of an HIV LTR for the RSV promoter, is described in Rosen, et al, PNAS 85:2071-2075 (1988). A control plasmid was also made using the same plasmid but wherein a stop codon was inserted in the vpr reading frame at a StyI site (35 amino acids). The plasmids do not contain a sufficient number of nucleotides corresponding to other portions of the HIV genome to express other HIV proteins.

A plasmid based on PSV₂CAT containing the CAT gene as described in Gorman, et al., Mol. Cell. Biol. 2:1044 (1982) was prepared. See lower portion of Figure 7. This plasmid contained SV40 polyadenylation signals and an enhancer. The enhancer-promoter used was replaced as indicated in Table 1. In one instance, it did include the SV40 enhancer promoter described in Gorman et al. The following enhancer-promoters were inserted into the plasmid at this site by using standard techniques: HIV-1, HIV-2, SL3, RSV, HTLV-1, CMV, cfos, IL-2-R-Hyb.

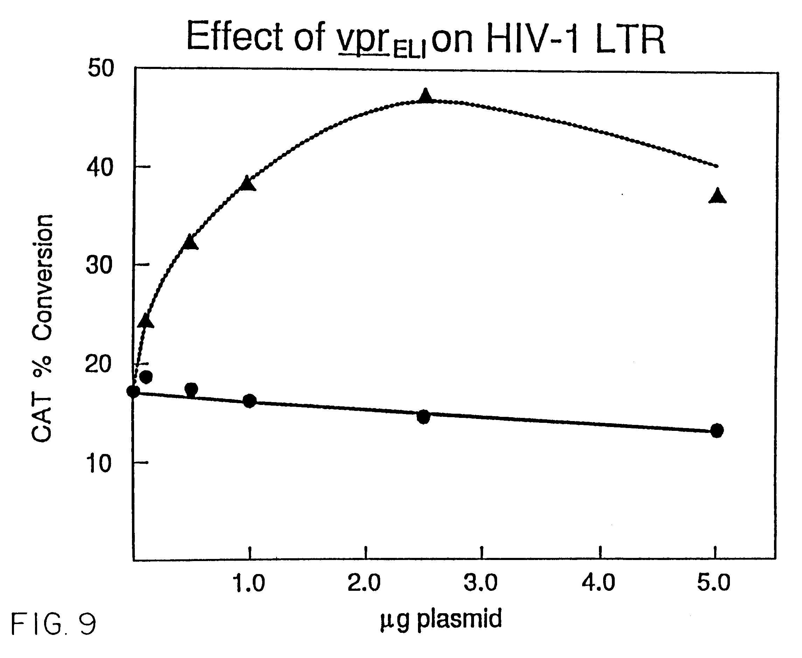

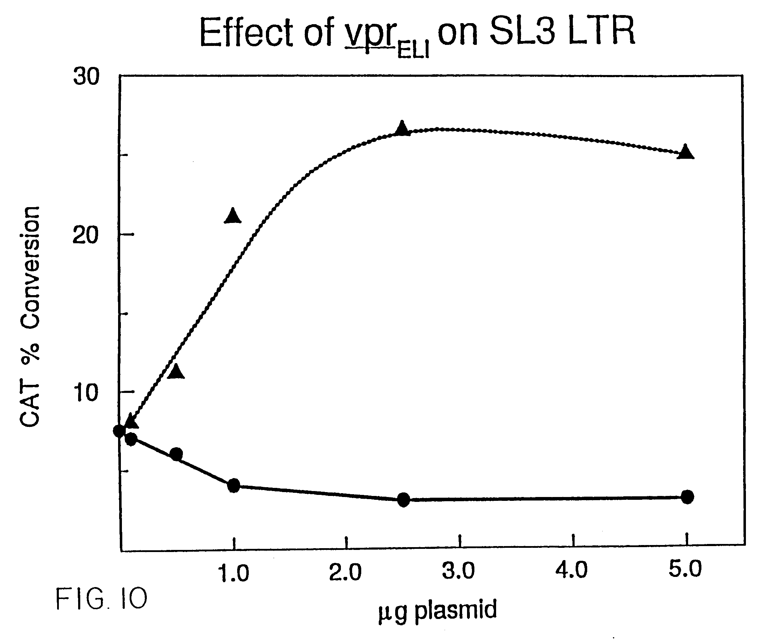

The transactivating ability of the vpr₊ gene was determined as follows. Jurkat cells were cotransfected by the DEAE-dextran method with 10µg of one of the CAT plasmids and either 10µg of the plasmid containing vpr⁺ gene (R expressor plasmid) or 10µg of the plasmid containing the vpr⁻ gene (control plasmid). 48 hours after transfection, cell lysates were assayed for CAT enzyme activity, as described in Gorman, C.M. et al. Mol. Cell. Biol. 2:, supra and Sodroski, J. et al, Science 225:381-384 (1984) (both of which are incorporated herein by reference). As shown by the data in Table 1, the vpr vectors enhanced the expression of CAT. The expression of the HIV LTR-directed CAT gene increased 3 to 4 fold in the presence of active vpr gene, as compared to the control expressor. See Figure 9. The triangles indicate Jurkat cells transfected with the R expressor plasmid, whereas the line with circles indicates Jurkat cells transfected with the control plasmid. The same increase of CAT expression was found when the above procedure was carried out in Jurkat cells stably producing the tat gene. The vpr and tat activation was additive. The expression of the SL₃ LTR-directed CAT gene increased 8 to 10 fold in the presence of active vpr gene, as compared to the control. See Figure 10. In Figure 10, the line with the triangles indicates the percent CAT conversion in Jurkat cells transfected with the R expressor plasmid whereas the line with the circles represents the Jurkat cells transfected with the control plasmid.

## Claims

1. A vector containing:
(a) a nucleotide segment corresponding to an active vpr gene of an HIV genome capable of expressing a protein having the ability to transactivate, wherein the vector does not contain the entire HIV genome; and
(b) a promoter upstream of the nucleotide segment.

2. The vector of claim 1, wherein the nucleotide segment corresponds to the vpr gene of the HIV strains ELI, MAL, and BRU.

3. The vector of claim 1, wherein the nucleotide segment is DNA.

4. The vector of claim 1, wherein the nucleotide segment is RNA.

5. The vector of claim 1, wherein the nucleotide segment consists essentially of the nucleotides corresponding to the vpr gene.

6. A DNA segment containing an active vpr gene that will express a vpr protein having a transactivating function, but wherein the DNA segment does not contain the entire HIV genome.

7. The DNA segment of claim 6, wherein the DNA segment consists essentially of the active vpr gene.

8. The vector of claim 2, wherein the promoter is a viral promoter and the vector also contains an enhancer and polyadenylation sequences.

9. A purified recombinant protein having transactivating ability produced by the active vpr gene of claim 6.

10. A protein produced by the nucleotide segment of claim 1.

11. The protein of claim 9, wherein the protein has a molecular weight of about 15kD.

12. An antibody that reacts specifically with the protein of claim 11, and can distinguish between active vpr and non-active vpr.

13. An immunogenic oligopeptide that corresponds to the C-terminal portion of the protein of claim 9 and is capable of producing an antibody, that can distinguish between active vpr and non-active vpr.

14. An antibody generated by the immunogenic oligopeptide of claim 13.

15. A method of assay for the presence of active viral protein R, which comprises:
(a) taking a predetermined sample;
(b) adding the antibody of claim 14; and
(c) determining, whether from the predetermined sample, a complex is formed with the antibody.

16. A cell line transformed by the vector of claim 1.

17. A method of producing high levels of a desired heterologous gene produce comprising:
(a) transfecting the cell line of claim 16 with a vector containing a preselected heterologous gene capable of expressing the desired heterologous gene product; and
(b) culturing the cell in a growth medium.

18. A method of producing high levels of a desired heterologous gene product comprising:
(a) cotransfecting a preselected cell with a first vector containing a predetermined heterologous gene capable of expressing the desired gene product, and a second vector, which second vector is the vector of claim 1 to transform the preselected cell; and
(b) culturing the transformed preselected cell in a growth medium.

19. A method of producing high levels of a desired heterologous gene product comprising:
(a) transfecting a cell expressing a desired heterologous gene product with the vector of claim 1; and
(b) culturing the cell in a growth medium.

20. A method of producing high levels of a desired heterologous gene product comprising;
(a) transfecting a cell with a vector containing a preselected heterologous gene capable of expressing the desired heterologous gene product; and
(b) culturing the cell in a growth medium supplemented with the protein of claim 11.

## Patentansprüche

1. Vektor, enthaltend:
(a) ein Nukleotidsegment, das einem aktiven vpr-Gen eines HIV-Genoms entspricht, welches in der Lage ist, ein Protein zu exprimieren, das die Fähigkeit zur trans-Aktivierung hat, wobei der Vektor nicht das gesamte HIV-Genom enthält; und
(b) einen Promotor stromaufwärts des Nukleotidsegments.

2. Vektor nach Anspruch 1, bei welchem das Nukleotidsegment dem vpr-Gen der HIV-Stämme ELI, MAL und BRU entspricht.

3. Vektor nach Anspruch 1, bei welchem das Nukleotidsegment DNA ist.

4. Vektor nach Anspruch 1, bei welchem das Nukleotidsegment RNA ist.

5. Vektor nach Anspruch 1, bei welchem das Nukleotidsegment im wesentlichen aus den dem vpr-Gen entsprechenden Nukleotiden besteht.

6. DNA-Segment, welches ein aktives vpr-Gen enthält, das ein vpr-Protein exprimiert, das eine trans-aktivierende Funktion hat, wobei das DNA-Segment jedoch nicht das gesamte HIV-Genom enthält.

7. DNA-Segment nach Anspruch 6, bei welchem das DNA-Segment im wesentlichen aus dem aktiven vpr-Gen besteht.

8. Vektor nach Anspruch 2, bei welchem der Promotor ein viraler Promotor ist und der Vektor ferner einen Enhancer und Polyadenylierungssequenzen enthält.

9. Gereinigtes rekombinantes Protein, das eine durch das aktive vpr-Gen von Anspruch 6 erzeugte trans-aktivierende Fähigkeit hat.

10. Protein, erzeugt durch das Nukleotidsegment von Anspruch 1.

11. Protein nach Anspruch 9, wobei das Protein ein Molekulargewicht von etwa 15 kD hat.

12. Antikörper, der spezifisch mit dem Protein von Anspruch 11 reagiert und zwischen aktivem vpr und nicht aktivem vpr unterscheiden kann.

13. Immunogenes Oligopeptid, das dem C-terminalen Abschnitt des Proteins von Anspruch 9 entspricht und in der Lage ist, einen Antikörper zu erzeugen, der zwischen aktivem vpr und nicht aktivem vpr unterscheiden kann.

14. Antikörper, erzeugt durch das immunogene Oligopeptid von Anspruch 13.

15. Verfahren zur Untersuchung auf die Anwesenheit von aktivem viralen Protein R, umfassend:
(a) Nehmen einer vorbestimmten Probe;
(b) Zugeben des Antikörpers von Anspruch 14; und
(c) Bestimmen, ob aus der vorbestimmten Probe ein Komplex mit dem Antikörper gebildet wird.

16. Zellinie, transformiert durch den Vektor von Anspruch 1.

17. Verfahren zur Erzeugung hoher Spiegel eines gewünschten heterologen Genprodukts, umfassend:
(a) Transfizieren der Zellinie von Anspruch 16 mit einem Vektor, der ein vorausgewähltes heterologes Gen enthält, das in der Lage ist, das gewünschte heterologe Genprodukt zu exprimieren; und
(b) Züchten der Zelle in einem Nährmedium.

18. Verfahren zur Erzeugung hoher Spiegel eines gewünschten heterologen Genprodukts, umfassend:
(a) Cotransfizieren einer vorausgewählten Zelle mit einem ersten Vektor, der ein vorbestimmtes heterologes Gen enthält, das in der Lage ist, das gewünschte Genprodukt zu exprimieren, und einem zweiten Vektor, welcher zweite Vektor der Vektor von Anspruch 1 ist, um die vorausgewählte Zelle zu transformieren; und
(b) Züchten der transformierten vorausgewählten Zelle in einem Nährmedium.

19. Verfahren zur Erzeugung hoher Spiegel eines gewünschten heterologen Genprodukts, umfassend:
(a) Transfizieren einer Zelle, die ein gewünschtes heterologes Genprodukt exprimiert, mit dem Vektor von Anspruch 1; und
(b) Züchten der Zelle in einem Nährmedium.

20. Verfahren zur Erzeugung hoher Spiegel eines gewünschten heterologen Genprodukts, umfassend:
(a) Transfizieren einer Zelle mit einem Vektor, der ein vorausgewähltes heterologes Gen enthält, das in der Lage ist, das gewünschte heterologe Genprodukt zu exprimieren; und
(b) Züchten der Zelle in einem Nährmedium, das mit dem Protein von Anspruch 1 ergänzt ist.

## Revendications

1. Vecteur contenant:
(a) un segment nucléotidique correspondant à un gène vpr actif d'un génome du HIV capable d'exprimer une protéine qui a une fonction transactivatrice, dans lequel
le vecteur ne contient pas l'intégralité du génome du HIV; et
(b) un promoteur en amont du segment nucléotidique.

2. Vecteur selon la revendication 1, dans lequel le segment nucléotidique correspond au gène vpr des souches du HIV ELI, MAL, et BRU.

3. Vecteur selon la revendication 1, dans lequel le segment nucléotidique est de l'ADN.

4. Vecteur selon la revendication 1, dans lequel le segment nucléotidique est de l'ARN.

5. Vecteur selon la revendication 1, dans lequel le segment nucléotidique est constitué essentiellement des nucléotides correspondant au gène vpr.

6. Segment d'ADN contenant un gène actif vpr capable d'exprimer une protéine vpr qui a une fonction transactivatrice, mais dans lequel le segment d'ADN ne contient pas l'intégralité du génome du HIV.

7. Segment d'ADN selon la revendication 6, dans lequel le segment d'ADN est constitué essentiellement du gène actif vpr.

8. Vecteur selon la revendication 2, dans lequel le promoteur est un promoteur viral et le vecteur contient également des séquences activatrices et de polyadénylation.

9. Protéine recombinante purifiée dotée d'une fonction transactivatrice produite par le gène vpr actif selon la revendication 6.

10. Protéine produite par le segment nucléotidique selon la revendication 1.

11. Protéine selon la revendication 9, dans laquelle la protéine présente un poids moléculaire d'environ 15 kDa.

12. Anticorps qui réagit spécifiquement avec la protéine selon la revendication 11 et peut distinguer entre la vpr active et la vpr inactive.

13. Oligopeptide immunogène qui correspond à la partie C-terminale de la protéine selon la revendication 9 et qui est capable d'induire la production d'un anticorps capable de distinguer entre la vpr active et la vpr inactive.

14. Anticorps produit en réponse à l'oligopeptide immunogène selon la revendication 13.

15. Procédé pour déterminer la présence de la protéine virale active R, qui comprend:
(a) le prélèvement d'un échantillon prédéterminé;
(b) l'adjonction de l'anticorps selon la revendication 14; et
(c) la détermination de la formation d'un complexe liant l'anticorps dans l'échantillon prédéterminé.

16. Lignée cellulaire transformée par le vecteur selon la revendication 1.

17. Procédé de production de quantités élevées du produit d'un gène hétérologue recherché comprenant:
(a) la transfection de la lignée cellulaire selon la revendication 16 avec un vecteur contenant un gène hétérologue présélectionné capable d'exprimer le produit du gène hétérologue recherché; et
(b) la mise en culture de la cellule dans un milieu de croissance.

18. Procédé de production de quantités élevées du produit d'un gène hétérologue recherché comprenant:
(a) la co-transfection d'une cellule présélectionnée avec un premier vecteur contenant un gène hétérologue prédéterminé capable d'exprimer le produit du gène recherché, et un second vecteur, lequel second vecteur est un vecteur selon la revendication 1 pour transformer la cellule présélectionnée ; et
(b) la mise en culture de la cellule présélectionnée transformée dans un milieu de croissance.

19. Procédé de production de quantités élevées du produit d'un gène hétérologue recherché comprenant:
(a) la transfection d'une cellule exprimant le produit d'un gène hétérologue recherché avec le vecteur selon la revendication 1; et
(b) la mise en culture de la cellule dans un milieu de croissance.

20. Procédé de production de quantité élevées du produit d'un gène hétérologue recherché comprenant:
(a) la transfection d'une cellule avec un vecteur contenant un gène hétérologue présélectionné capable d'exprimer le produit du gène hétérologue recherché; et
(b) la mise en culture de la cellule dans un milieu de croissance supplémenté en la protéine selon la revendication 11.
